# EUROPÄISCHE PATENTSCHRIFT

(11) **EP 1 175 871 B1**
(45) Veröffentlichungstag und Bekanntmachung des Hinweises auf die Patenterteilung: **18.11.2009**
(21) Anmeldenummer: 01115995.1
(22) Anmeldetag: 30.06.2001
(51) Int. Cl.: A61B 17/72, A61B 17/17

(54) **Verriegelungsnagel**
Locking nail
Clou de verrouillage

(30) Priorität: 26.07.2000 DE 20012877 U
(43) Veröffentlichungstag der Anmeldung: 30.01.2002
(73) Patentinhaber: Stryker Trauma GmbH, 24232 Schönkirchen (DE)
(72) Erfinder: Robioneck, Bernd, 24211 Preetz (DE)
(74) Vertreter: Kopf, Korbinian Paul

(56) Entgegenhaltungen:
- DE-U- 9 401 916

## Beschreibung

Die Erfindung bezieht sich auf einen Verriegelungsnagel nach dem Obergriff des Anspruchs 1.

Verriegelungsnägel zur Versorgung von Knochenbrüchen sind seit längerem bekannt. Sie werden in den vorbereiteten bzw. aufgebohrten Knochenkanal intramedullär eingeführt, bei Femurbrüchen von proximal oder distal und im Knochen verriegelt. Die Verriegelung geschieht dadurch, dass durch Querbohrungen an den Enden des Nagelschaftes Knochenschrauben geführt werden. Dadurch hält der Verriegelungsnagel die Knochenteile axial zusammen und hindert sie auch an der Rotation.

Derartige Verriegelungsnägel sind etwa aus US 4,622,959 bekannt geworden. Es ist ferner bekannt, derartige Verriegelungsnägel für die Führung und Halterung von Schenkelhalsschrauben einzusetzen, wie etwa aus EP 0 321 170 oder G 85 28 770 U1 bekannt geworden.

Es ist schliesslich auch bekannt, derartige Verriegelungsnägel mit einer Kompressionsmöglichkeit zu versehen, wie aus DE 3 541 597, der US 4,281,649 oder der DE 94 01 916 U1 bekannt geworden. Zumeist das proximale Ende des Verriegelungsnagels ist mit einer länglichen Querbohrung versehen, und im hohlen Endabschnitt des Nagelschaftes ist ein Innengewinde vorgesehen, für die Aufnahme einer Kompressionsschraube, mit deren Hilfe die Knochenschraube in der länglichen Querbohrung parallel zu ihrer Achse verstellt werden kann.

Der Erfindung liegt die Aufgabe zugrunde, einen Verriegelungsnagel der eingangs genannten Art dahingehend zu verbessern, dass er trotz seiner Kompressionseigenschaft verbesserte Verriegelungseigenschaften aufweist.

Diese Aufgabe wird durch die Merkmale des Anspruchs 1 gelöst.

Bei dem erfindungsgemäßen Verriegelungsnagel ist zwischen der länglichen Querbohrung und dem zugekehrten Ende des Nagelschaftes eine weitere Querbohrung für eine Knochenschraube vorgesehen. Ferner liegt das Innengewinde für das Verriegelungselement zwischen der länglichen und der weiteren Querbohrung, und der Abstand der Bohrungen und die Länge des Verriegelungselements sind so gewählt, daß das Verriegelungselement ein Durchführen einer Knochenschraube durch die weitere Querbohrung auch dann ermöglicht, wenn die Knochenschraube sich an dem Ende der länglichen Querbohrung befindet, welches dem Ende des Nagelschaftes bzw. der weiteren Querbohrung zugekehrt ist. Nach dem Einsetzen des Nagels und dem Auffinden der Querbohrungen im Knochenkanal mit Hilfe eines Zielgeräts wird auch die Knochenschraube für die längliche Querbohrung eingesetzt. Zweckmäßigerweise ist ihre Lage derart, daß anschließend mit Hilfe des Verriegelungselements die Knochenschraube in der länglichen Querbohrung parallel zu sich selbst verstellt werden kann, wodurch eine Komprimierung an der Bruchstelle bewirkt wird. Nachdem dieser Vorgang beendet ist, kann dann durch die weitere Querbohrung eine weitere Knochenschraube geführt und der Nagel entsprechend verriegelt werden.

Die weitere Querbohrung läßt zum Ende des Nagelschaftes ausreichend Platz für ein weiteres Innengewinde, in das das Zielgerät und/oder ein Eintreib- und Ausziehinstrument eingeschraubt werden kann.

Aus der bereits genannten EP 0 321 170 ist bereits bekannt, am Nagelschaft eines Verriegelungsnagels für die Führung einer Schenkelhalsschraube zwei Gewindeabschnitte vorzusehen. Der weiter innen liegende Gewindeabschnitt dient für eine Sicherungsschraube, durch welche die Schenkelhalsschraube an einer Drehung gehindert wird. Der äußere Gewindeabschnitt dient der Aufnahme des Zielgeräts bzw. eines Eintreib- oder Ausziehinstruments.

Nach einer Ausgestaltung der Erfindung für einen Tibianagel ist die Achse der weiteren Querbohrung um einen Winkel von vorzugsweise 90° gegenüber der Achse der länglichen Querbohrung versetzt.

Die Erfindung wird nachfolgend anhand eines in Zeichnungen dargestellten Ausführungsbeispiels näher erläutert.
- Fig. 1: zeigt perspektivisch das Ende eines Tibia-Verriegelungsnagels, teilweise aufgebrochen.
- Fig. 2: zeigt eine ähnliche Ansicht wie Fig. 1, jedoch nach erfolgter Kompression und Einsetzens einer weiteren Knochenschraube durch eine weitere Querbohrung.

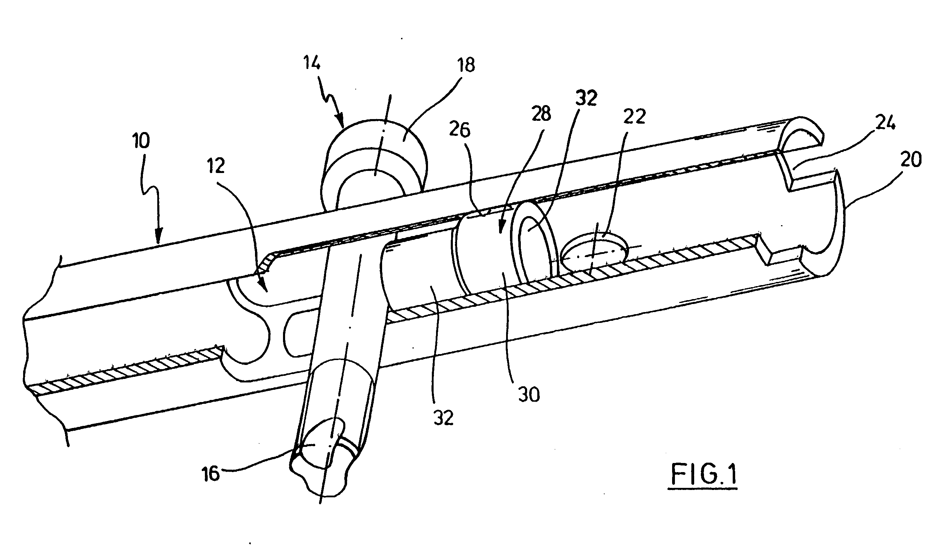

In Figuren 1 uns 2 ist der Endabschnitt 10 eines Verriegelungsnagels dargestellt (Je nach Anwendung handelt es sich um das proximale oder distale Ende des Nagels, wobei dieses jeweils auch das Anschlußende eines Zielgerätes und/oder eines Einschlaginstruments ist). Wie erkennbar, ist der Nagelabschnitt 10 hohl. Auch der nicht gezeigte Rest des Verriegelungsnagels kann hohl ausgeführt sein. Im nicht gezeigten distalen Abschnitt weist der Verriegelungsnagel ein, zwei oder drei Querbohrungen auf, wie an sich bekannt. Im Abschnitt 10 weist der Verriegelungsnagel eine längliche Querbohrung 12 auf, durch welche hindurch eine Knochenschraube 14 geführt ist mit einem selbstschneidendem Gewindeabschnitt 16 und einem Kopf 18 mit versenkten Schlüsselflächen (nicht zu erkennen). Im Abstand zum hier proximalen Ende 20 des Endabschnitts 10 und zwischen dem Ende 20 und der länglichen Bohrung 12 ist eine weitere Querbohrung 22 vorgesehen, deren Achse annähernd senkrecht zur Achse der länglichen Querbohrung 12 verläuft. Das proximale Ende 20 ist mit gegenüberliegenden Aussparungen 24 versehen, die in Eingriff bringbar sind mit entsprechenden Vorsprüngen eines nicht gezeigten Zielgerätes oder eines Eintreib- oder Ausziehinstruments. Letztere erhalten durch die Aussparungen 24 eine vorgegebene Drehlage zum Verriegelungsnagel.

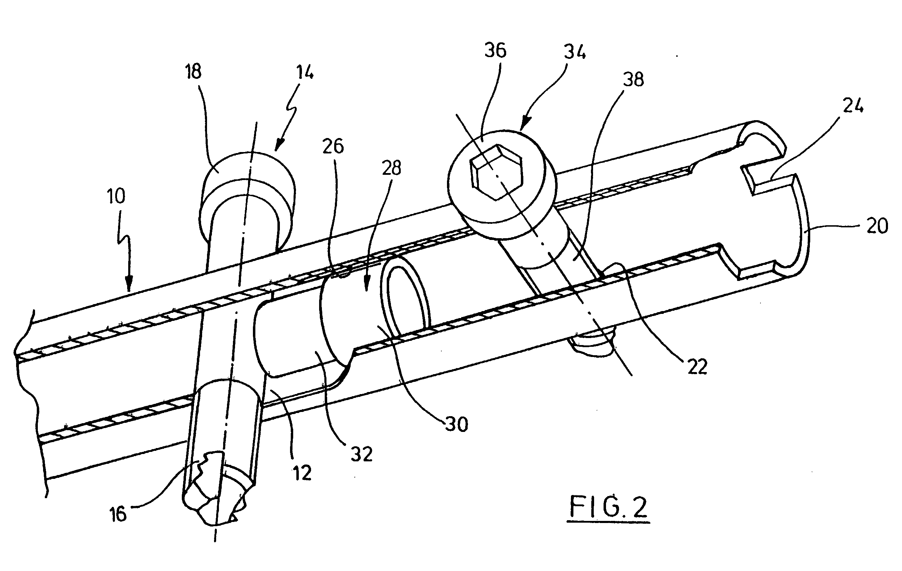

Zwischen den Querbohrungen 12 und 22 ist im Kanal des Nagelabschnitts 10 ein Innengewindeabschnitt 26 geformt, und zwischen den Querbohrungen 12, 22 ist ein Verriegelungselement 28 angeordnet mit einem Gewindeabschnitt 30 und einem gewindefreien Abschnitt 32 von kleinerem Durchmesser. Die Länge des Verriegelungselements 28 ist so bemessen, daß es auch dann nicht in den Bereich der weiteren Querbohrung 22 reicht, wenn, wie in Fig. 1 dargestellt, die Knochenschraube 14 sich an dem Ende der länglichen Querbohrung 12 befindet, welche der weiteren Querbohrung 22 zugekehrt ist. Der Gewindeabschnitt ist mit dem Innengewinde 26 in Eingriff. Am rechten Ende weist das Verriegelungselement 28 versenkte Eingriffsflächen 32 (Innensechskant) für ein Werkzeug auf, so daß es im Nagelkanal verstellt werden kann, wie dies aus Fig. 2 zu erkennen ist. In Fig. 2 ist gezeigt, wie mit Hilfe des Verriegelungselements 28 die Knochenschraube 14 bis zum anderen Ende der länglichen Querbohrung 12 parallel zu sich selbst verstellt worden ist. Bei einem herkömmlichen Einsatzfall wird dies selten eintreten. Vielmehr wird die Knochenschraube 14 lediglich über einen Bruchteil der Länge der länglichen Querbohrung 12 verstellt werden.

Ist die Kompression beendet, kann eine weitere Knochenschraube 34 mit Kopf 36 und Gewindeschaft 38 durch die weitere Querbohrung 22 hindurchgeführt werden, um das Ende des Nagels zu verriegeln.

Wie schon erwähnt, ist üblich, ein Eintreibinstrument oder auch das Zielgerät für den Verriegelungsnagel auf das zugeordnete Ende des Nagels aufzusetzen und zu verschrauben. Es ist nun von Vorteil, wenn während des Kompressionsvorgangs das Zielgerät auf dem Nagelende verbleiben kann. Der Kompressionsvorgang erfolgt dadurch, daß mit Hilfe eines Schraubendrehers oder dergleichen das Verriegelungselement im zugeordneten Gewinde verdreht wird. Hierzu wird der Schaft des Schraubendrehers in das hohle Ende des Nagels eingeführt. Zuvor ist die Schraube, mit der das Zielgerät am Nagelende festgelegt worden ist, entfernt worden. Bei der Komprimierung verbleibt jedoch, wie erwähnt, das Zielgerät an dem Nagelende. Der Schaft des Schraubendrehers wird dabei durch den Abschnitt hindurchgeführt, in dem sonst die Schraube zur Befestigung des Zielgeräts am Nagelende einsitzt. Ist der Kompressionsvorgang beendet, wird der Griff vom Schraubendreher entfernt. Der Schaft des Schraubendrehers ist nun so ausgeführt, und die hohle Schraube oder Mutter zur Befestigung des Zielgerätes so bemessen, daß sie über den Schraubendreherschaft übergeschoben werden kann. Dadurch kann das Zielgerät wieder am Nagel verschraubt werden bei stehen gebliebenem Schraubendreherschaft. Dieser hat mithin eine Führungsfunktion für das Wiederanschrauben des Zielgerätes. Danach kann der Schaft durch einfaches Abziehen entfernt werden. Nunmehr ist das Zielgerät wiederum fest mit dem Nagel verbunden, und die Lage der weiteren Querbohrung kann in bekannter Weise ermittelt werden. Ist dies geschehen, erfolgt dann die Anbringung einer Knochenschraube in der weiteren Querbohrung in an sich bekannter Weise.

Der Aufbau des Zielgeräts ist hierbei völlig nebensächlich. Wesentlich ist nur, daß die Anbringung des Zielgerätes die beschriebene Funktion gewährleistet.

## Patentansprüche

1. Verriegelungsnagel mit
einem Schaft (10), welcher ein erstes Ende (20) mit einem hohl ausgeführten Schaftabschnitt, und ein zweites Ende aufweist,
einer länglichen Querbohrung (12) nahe dem ersten Ende (20), welche in Längsrichtung des Schafts länglich geformt ist, und
einer Querbohrung nahe dem zweiten Ende für die Aufnahme jeweils einer Knochenschraube,
einem Innengewinde (26), welches in dem hohl ausgeführten Schaftabschnitt ausgebildet ist, und
einem Verriegelungselement mit einem Außengewindeabschnitt, das in das Innengewinde (26) einschraubbar ist und mit der Knochenschraube in der länglichen Querbohrung (12) zusammenwirken kann, um diese in der länglichen Querbohrung quer zu ihrer Längsachse zu verstellen, wobei
zwischen der länglichen Querbohrung (12) und dem ersten Ende (20) des Schaftes (10) eine weitere Querbohrung (22) für eine Knochenschraube (34) vorgesehen ist, **dadurch gekennzeichnet, dass**
die Länge des Verriegelungselements (28) und der Abstand der länglichen und der weiteren Querbohrung (22) so bemessen sind, dass das mit der Knochenschraube in der langlichen Querbohrung zusammenwirkende Verriegelungselement (28) die weitere Querbohrung (22) frei lässt und nicht in den Bereich der weiteren Querbohrung reicht, selbst dann, wenn die in der länglichen Querbohrung (12) befindliche Knochenschraube (14) an dem dem ersten Ende (20) zugekehrten Ende der länglichen Querbohrung (12) anliegt.

2. Verriegelungsnagel nach Anspruch 1, **dadurch gekennzeichnet, dass** die Achse der weiteren Querbohrung (22) um einen Winkel von vorzugsweise 90° gegenüber der Achse der länglichen Querbohrung (12) versetzt ist.

3. Verriegelungsnagel nach Anspruch 1 oder 2, **dadurch gekennzeichnet, dass** das Verriegelungselement (28) aus dem Außengewindeabschnitt (30) und einem gewindefreien Abschnitt (32) besteht, der mit der Knochenschraube (14) in der länglichen Querbohrung (12) zusammenwirken kann.

4. Verriegelungsnagel nach einem der Ansprüche 1 bis 3, **dadurch gekennzeichnet, dass** ein weiterer Innengewindeabschnitt für ein Ziel- und/oder ein Eintreib- und Ausziehinstrument im hohlen Schaftabschnitt des Schaftes (10) zwischen der weiteren Querbohrung (22) und dem ersten Ende (20) des Schaftes (10) vorgesehen ist.

5. Kombination eines Verriegelungsnagels nach einem der Ansprüche 1 bis 4 mit einem Instrument, **dadurch gekennzeichnet, dass** das Instrument einen Griff und einen länglichen Schaft des Instruments aufweist, wobei der Schaft des Instruments mit dem ersten Ende (20) des Verriegelungselements (28) in Eingriff bringbar ist, dass der Griff vom Schaft des Instruments lösbar ist, und dass eine Befestigungsschraube für ein Zielgerät am Verriegelungsnagel so geformt ist und der Durchmesser des Schaftes des Instrumentes so bemessen ist, dass die Befestigungsschraube über den länglichen freien Schaft des Instruments geschoben werden kann zur Befestigung des Zielgeräts an dem Verriegelungsnagel bei in den Verriegelungsnagel eingeführtem bzw. einsitzendem Schaft des Instruments.

## Claims

1. Interlocking nail with
a shaft (10) which has a first end (20) with a hollow shaft section and a second end,
a longitudinal transverse hole (12) close to the first end (20), which is longitudinally shaped in the longitudinal direction of the shaft, and
a transverse hole close to the second end for accommodating one bone screw each,
an internal thread (26) provided in the hollow shaft section, and
a locking element with an external threaded section which can be screwed into the internal thread (26) and can interact with the bone screw in the longitudinal transverse hole (12) in order to adjust it in the longitudinal transverse hole transversely to its longitudinal axis, wherein
between the longitudinal transverse hole (12) and the first end (20) of the shaft (10) a further transverse boring (22) for a bone screw (34) is provided, **characterised in that** the length of the locking element (28) and the distance between the longitudinal and further transverse hole (22) are such that the locking element (28) interacting with the bone screw in the longitudinal transverse hole leaves the further transverse hole (22) free and does not extend into the area of the further transverse hole, even if the bone screw (14) located in the longitudinal transverse hole (12) is in contact with the end of the longitudinal transverse hole (12) facing the first end (20).

2. Interlocking nail in accordance with claim 1, **characterised in that** the axis of the further transverse hole (22) is offset by an angle of preferably 90° vis-à-vis the axis of the longitudinal hole (12).

3. Interlocking nail in accordance with claim 1 or 2 **characterised in that** the locking element (28) comprises the external thread section (30) and a thread-free section (32) which can interact with the bone screw (14) in the longitudinal transverse hole (12).

4. Interlocking nail in accordance with any one of claims 1 to 3 **characterised in that** a further internal thread section for an aiming and/or insertion and withdrawal instrument is provided in the hollow shaft section of the shaft (10) between the further transverse hole (22) and the first end (20) of the shaft (10).

5. Combination of an interlocking nail in accordance with any one of claims 1 to 4 with an instrument, **characterised in that** the instrument has a grip and a longitudinal shaft of the instrument, wherein the shaft of the instrument can be brought into engagement with the first end (20) of the locking element (28), that the grip can be detached from the shaft of the instrument and that a fastening screw for an aiming device is formed at the interlocking nail in such a way and the diameter of the shaft of the instrument is dimensioned in such a way that the fastening screw can be pushed over the longitudinal free shaft of the instrument to fasten the aiming device on the interlocking nail when the shaft of the instrument is inserted and/or located in the interlocking nail.

## Revendications

1. Clou de verrouillage comprenant
une tige (10), qui présente une première extrémité (20) avec une section de tige de réalisation creuse, et une seconde extrémité,
un alésage transversal (12) oblong au voisinage de la première extrémité (20), qui est de forme allongée dans la direction longitudinale de la tige, et
un alésage transversal au voisinage de la seconde extrémité pour recevoir une vis pour os respective,
un taraudage (26) qui est réalisé dans la section de tige creuse, et
un élément de verrouillage avec une section de filetage extérieur, qui peut être vissé dans le taraudage (26) et coopérer avec la vis pour os dans l'alésage transversal oblong (12), afin de la déplacer dans l'alésage transversal oblong transversalement à son axe longitudinal,
un autre alésage transversal oblong (22) pour une vis pour os (34) étant prévu entre l'alésage transversal oblong (12) et la première extrémité (20) de la tige (10),
**caractérisé en ce que**
la longueur de l'élément de verrouillage (28) et l'écartement de l'alésage transversal oblong et de l'autre alésage transversal (22) sont dimensionnés de sorte que l'élément de verrouillage (28), coopérant avec la vis pour os dans l'alésage transversal oblong, laisse libre l'autre alésage transversal (22) et ne parvient pas dans la zone de l'autre alésage transversal, même lorsque la vis pour os (14), située dans l'alésage transversal oblong (12), s'applique sur l'extrémité de l'alésage transversal oblong (12) tournée vers la première extrémité (20).

2. Clou de verrouillage suivant la revendication 1, **caractérisé en ce que** l'axe de l'autre alésage transversal (22) est en déport d'un angle de préférence de 90° par rapport à l'axe de l'alésage transversal oblong (12).

3. Clou de verrouillage suivant l'une des revendications 1 ou 2, **caractérisé en ce que** l'élément de verrouillage (28) est constitué de la section de filetage extérieur (30) et d'une section (32) sans filetage, qui peut coopérer avec la vis pour os (14) dans l'alésage transversal oblong (12).

4. Clou de verrouillage suivant l'une des revendications 1 à 3, **caractérisé en ce qu'**une autre section taraudée est prévue pour un instrument de visée et/ou d'insertion et d'extraction dans la section creuse de la tige (10), entre l'autre alésage transversal (22) et la première extrémité (20) de la tige (10).

5. Combinaison d'un clou de verrouillage suivant l'une des revendications 1 à 4 avec un instrument, **caractérisée en ce que** l'instrument présente une poignée et une tige oblongue de l'instrument, la tige de l'instrument pouvant être amenée en prise avec la première extrémité (20) de l'élément de verrouillage (28), que la poignée est amovible de la tige de l'instrument, et qu'une vis de fixation pour un appareil de visée est formée sur le clou de verrouillage et le diamètre de la tige de l'instrument est dimensionné de sorte que la vis de fixation peut être poussée sur la tige libre oblongue de l'instrument pour la fixation de l'appareil de visée sur le clou de verrouillage, lorsque la tige de l'instrument est introduite et/ou placée dans le clou de verrouillage.
